# EUROPEAN PATENT SPECIFICATION

(11) **EP 2 611 378 B1**
(45) Date of publication and mention of the grant of the patent: **05.12.2018**
(21) Application number: 10856541.7
(22) Date of filing: 31.08.2010
(51) Int. Cl.: A61B 90/00, A61B 17/00, A61B 34/20, A61B 34/00, A61B 17/15

(54) **PROXIMITY-TRIGGERED COMPUTER-ASSISTED SURGERY SYSTEM**
NÄHERUNGSAUSGELÖSTES COMPUTERUNTERSTÜTZTES CHIRURGISCHES SYSTEM
SYSTÈME DE CHIRURGIE ASSISTÉE PAR ORDINATEUR DÉCLENCHÉ PAR LA PROXIMITÉ

(43) Date of publication of application: 10.07.2013
(73) Proprietor: Orthosoft, Inc., Montreal, Québec H3C 2N6 (CA)
(72) Inventor: BOUTIN, Yannick, Montréal Québec H2S 2K5 (CA); ABIVEN, Jean-Guillaume, Montréal Québec H3K 2K7 (CA); CHEVRIER, Mathieu, Roxboro Québec H8Y 1M1 (CA)
(74) Representative: Taor, Simon Edward William
(86) International application number: PCT/CA2010/001311
(87) International publication number: WO 2012/027815

(56) References cited:
- WO-A1-2009/063421
- US-A1- 2003 078 494
- US-A1- 2007 225 731
- US-A1- 2009 099 570
- US-A1- 2009 248 044
- US-B2- 6 946 956
- US-B2- 6 954 148

## Description

### FIELD OF THE APPLICATION

*The present application relates to computer-assisted surgery, and more particularly to surgical tools used in computer-assisted surgery and triggering associated therewith.*

### BACKGROUND OF THE ART

Computer-assisted surgery has evolved over the years to use the computational speed of computers to guide surgeons and operating-room personnel in performing orthopedic procedures on the bones with high degrees of precision and accuracy. In order to lessen the requirements of hardware in the operating room, micro-electromechanical systems (MEMS) such as gyroscopes and accelerometers are used in calculating orientation and/or position of surgical tools and bones.

MEMS are used in addition or as an alternative to other types of trackers, such as optical tracking systems (e.g., Navitrack™). In some cases, optical tracking systems can be replaced with MEMS, therefore removing bulky optical tracker devices on tools and bones. Because of the minute format of MEMS, it is even contemplated to perform computer-assisted surgery without a self-standing monitor, by instead providing all information within the surgical field with LED indicators or screens on tools. It is desirable to automate computer-assisted surgery using MEMS to accelerate surgical procedures.

International patent application WO 2009/063421 discloses a therapeutic ultrasound tracking system including a first and second plurality of tracking elements, a tracking generator, and a system controller. The system controller detects whether a therapeutic ultrasound probe is positioned within an allowable position and orientation with respect to a target region.

United States patent application US 2009/0248044 discloses a computer-assisted surgery system for planning/guiding alterations to a bone in surgery, comprising a trackable member adapted to be secured to the bone.

### SUMMARY OF THE APPLICATION

Therefore, in accordance with a first embodiment according to the invention, there is provided a computer-assisted surgery system comprising: a first surgical device with a tracking unit mounted thereon and tracked during a surgical procedure and adapted to perform a first function associated to the surgical procedure; a second surgical device adapted to perform a second function associated to the surgical procedure; a triggered unit triggered when the first surgical device and the second surgical device reach a predetermined proximity relation; a surgical procedure processing unit tracking at least the first surgical device, the surgical procedure processor comprising a trigger detector detecting a triggering of the triggered unit, a computer-assisted surgery application operating steps of a surgical procedure, a controller for commanding the computer-assisted surgery application to activate a selected step associated with the second function in the surgical procedure when the trigger detector signals a detection, and an interface for displaying information about the selected step in the surgical procedure.

Further in accordance with the invention, one of the surgical devices has a female connector, another of the surgical devices has a male connector, whereby the surgical devices interconnect in a mating engagement of the male and female connectors.

Still further in accordance with the first embodiment, the triggered unit is a switch and the predetermined proximity relation is the mating engagement, whereby the switch is triggered by the mating engagement of the male and the female connector.

Still further in accordance with the first embodiment, the first surgical device is a reference tool mounted to the bone for tracking of the bone.

Still further in accordance with the first embodiment, the second surgical device is a digitizing tool releasably connected to the reference tool for determining an axis of the bone.

Still further in accordance with the first embodiment, the system further comprises a conditions database in one of the triggered unit and the trigger detector to confirm that the predetermined proximity relation is in accordance with conditions of the conditions database.

Still further in accordance with the first embodiment, the next step is a change of mode, and the interface automatically changes mode when the trigger detector signals a detection.

Still further in accordance with the first embodiment, the second surgical device has a tracking unit for being tracked during the surgical procedure.

Still further in accordance with the first embodiment, the tracking unit comprises a micro-electromechanical system.

In accordance with a second embodiment not forming part of the invention, there is provided a method for progressing through steps of a surgical procedure of a computer-assisted surgery application, comprising: tracking at least a first surgical device adapted to perform a first function associated with the surgical procedure; detecting a predetermined proximity relation between the first surgical device and a second surgical device adapted to perform a second function associated with the surgical procedure; activating a selected step of the surgical procedure associated with the second function when the predetermined proximity relation is detected; and displaying information related to the selected step.

Further in accordance with the second embodiment, tracking the first surgical device comprises receiving tracking data from a micro-electromechanical system.

Still further in accordance with the second embodiment, detecting the predetermined proximity relation comprises detecting a mating engagement between the first surgical device and the second surgical device.

Still further in accordance with the second embodiment, the method further comprises tracking the second surgical device.

Still further in accordance with the second embodiment, tracking the second surgical device comprises tracking the second surgical device from the tracking of the first surgical device and from a known geometry of the mating engagement between the surgical devices.

Still further in accordance with the second embodiment, displaying information related to the selected step comprises displaying information related to the tracking of the second surgical device.

Still further in accordance with the second embodiment, the method comprises confirming that the detected predetermined proximity relation respects predetermined conditions prior to activating the selected step.

Still further in accordance with the second embodiment, tracking the first surgical device comprises tracking a tibial reference on a tibia of a patient.

Still further in accordance with the second embodiment, the method further comprises tracking the second surgical device with the second surgical device being a tibial digitizer, and wherein displaying information related to the selected step comprises displaying a tibial axis.

### BRIEF DESCRIPTION OF DRAWINGS

Fig. 1 is a block diagram of a proximity-triggered computer-assisted surgery system in accordance with an exemplary embodiment of the present application;
Fig. 2 is a schematic view of mating surgical devices with respect to a bone, as used with the computer-assisted surgery system of Fig. 1;
Fig. 3 is a schematic view of mating surgical devices with respect to a bone, as used with the computer-assisted surgery system of Fig. 1;
Fig. 4A is a schematic view of the mating surgical devices of Fig. 2 with a CAS monitor, prior to mating; and
Fig. 4B is a schematic view of the mating surgical devices of Fig. 2 with the CAS monitor, after mating.

### DESCRIPTION OF EXEMPLARY EMBODIMENTS

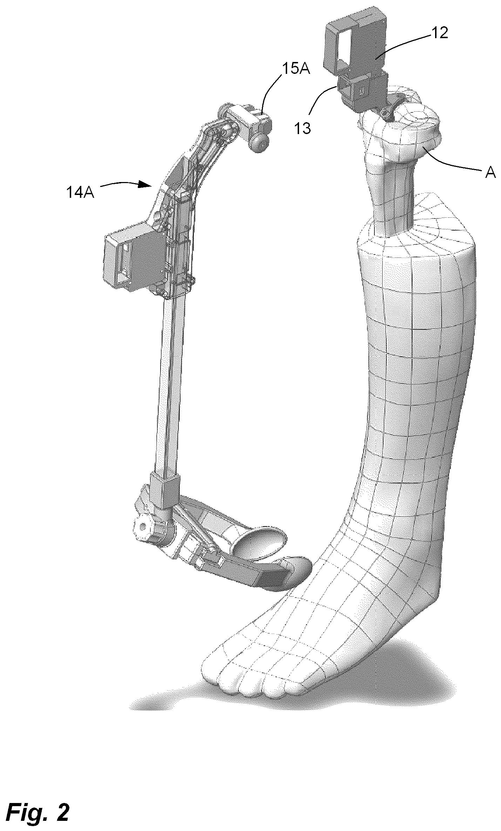

Referring to the drawings, and more particularly to Figs. 2, 3, 4A and 4B, there is illustrated a lower leg of a patient, with soft tissue removed so as to expose an upper end of the tibia A, for use with a proximity-triggered computer-assisted surgery system of the exemplary embodiment. Although the computer-assisted surgery system and associated method are described and illustrated as used for tibial alterations during knee-replacement surgery, it is understood that the proximity-triggered computer-assisted surgery system and method of the present embodiment may be used in all other types of orthopedic surgery, such as total knee replacement, total hip replacement, spine surgery, or any other type of orthopedic surgery requiring surgical devices as described for the exemplary embodiment.

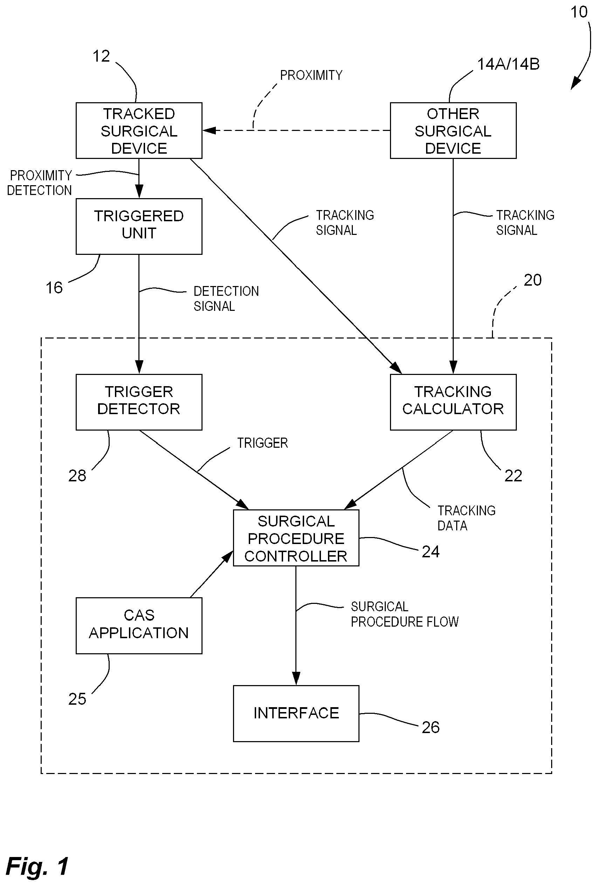

Referring to Fig. 1, the proximity-triggered computer-assisted surgery (CAS) system is generally shown at 10. The CAS system 10 may feature a plurality of surgical devices. In the illustrated embodiment, the CAS system 10 has a tracked surgical device 12 that is secured to a bone. In the exemplary embodiment of Fig. 1, the tracked surgical device 12 is a MEMS-operated reference unit that is secured to the bone so as to provide tracking data (i.e., orientation and/or position information) related to the bone A through various calibration and referencing steps.

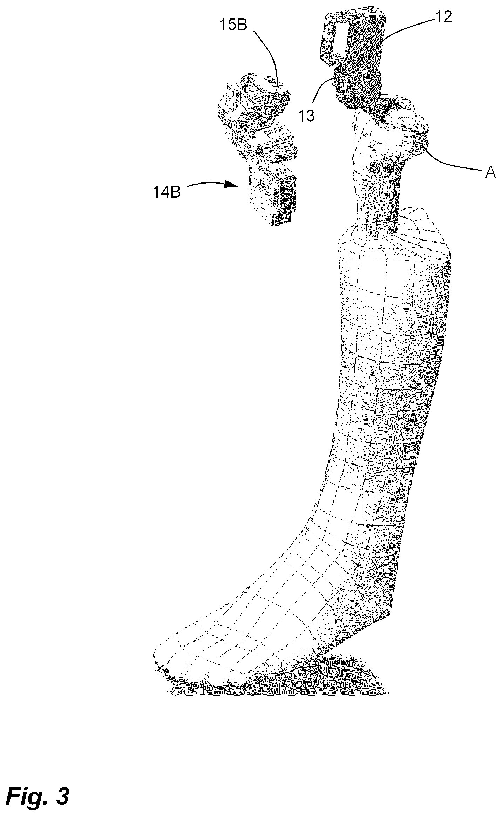

As shown in Figs. 2 and 3, the tracked surgical device 12 is a tibial reference secured to an upper end of the tibia A, so as to act as a tracking reference. The tibial reference 12 has a female connector 13 in order to receive other surgical devices therein. In one embodiment, other surgical devices in mating engagement with the tracked surgical device 12 will also be tracked by the geometrical relation between the tracked surgical device 12 and the other surgical device. In Fig. 2, the other surgical device is illustrated at 14A and is known as a tibial digitizer. In Fig. 3, the other surgical device 14B is a cutting block. The tibial digitizer and the cutting block respectively have male connectors 15A and 15B.

In the exemplary embodiment, the tracked surgical device 12 has electronic circuitry, as it operates MEMS to provide the tracking data. A triggered unit 16 (Fig. 1) is provided on the tracked surgical device 12, for instance as part of the electronic circuitry, so as to be triggered when a mating engagement is completed between the female connector 13 of the tracked surgical device 12 and the male connector 15 of the other surgical device 14. The triggered unit 16 may be any one of a plurality of units. For instance, the triggered unit 16 may be a simple switch that is provided in the female connector 13 so as to be triggered by the contact with the male connector 15. Other alternatives include a magnet and appropriate sensor, respectively in opposite devices, proximity switches and sensors, a conductive element and an open circuit in opposite devices, or any other appropriate type of triggered unit or switch.

The proximity-triggered CAS system 10 features a proximity-triggered CAS processing unit 20 that comprises a tracking calculator 22 in order to track the surgical device 12 and the surgical devices 14A/14B through the connected relation with the surgical device 12, or independently therefrom of the surgical device 14A/14B have their own MEMS. The tracking calculator 22 receives tracking signals from the MEMS of the surgical devices and converts the data with prior calibration and referencing information into tracking values related to the surgical devices 12, 14A and/or 14B, as well as related to the bone A or any other appropriate bone that has been calibrated and referenced as well.

A surgical procedure controller 24 operates a CAS application 25 that guides the surgeon and personnel of the operating room in following a series of manual steps according to the CAS application to define bone axes, tool axes, models, as well as in providing surgical step information, to guide surgical operations on the bones. The CAS application 25 follows a specific flow of steps according to the information entered by the operator of the CAS system 10, as well as through the tracking data provided by the tracking calculator 22. The resulting information is displayed on an interface 26, typically a monitor of a self-standing station, or screen or LED indicators directly on the surgical devices.

A trigger detector 28 is provided in the CAS processing unit 20 so as to receive a detection signal from the triggered unit 16. Upon receiving the detection signal, the trigger detector 28 signals the triggering to the surgical procedure controller 24. The trigger detector 28 may perform a confirmation step, for instance by confirming that the surgical device 14 is sufficiently close to the surgical device 12, when proximity sensors are used. Moreover, if the devices 12 and 14 matingly engage, the trigger detector 28 may require a sustained detection signal to confirm the triggering. These confirmation steps may be performed by the triggered unit 16 as well, in both case by the presence of a conditions database.

The triggering is automatic further to the positioning of the surgical device 14 in proximity to or in contact with the surgical device 12. Accordingly, the surgical procedure controller 24 will alter its flow of operations following the receipt of a signal from the trigger detector 28 to further advance the flow of steps of the surgical procedure, as observed on the interface 26, for instance by the change of data on the screen. Accordingly, by performing this action, one step of interfacing between the operator and the CAS system 10 is removed, and replaced by an intuitive step required in most standard surgical techniques for a given type of procedure.

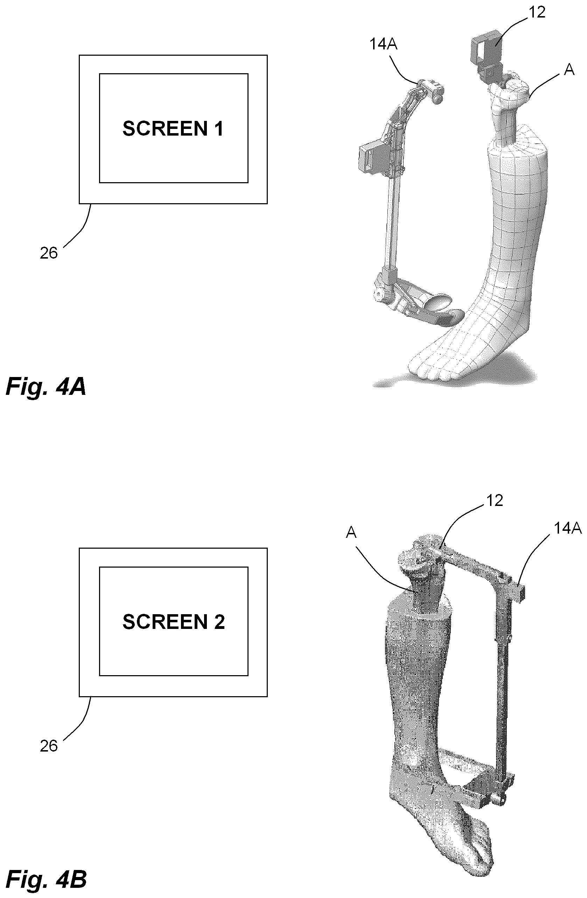

Referring to Figs. 4A and 4B, there is illustrated the method of automatically triggering the CAS system by proximity. In Fig. 4A, the tibial reference 12 is secured to the tibia A, and is calibrated and referenced. Accordingly, the tibia A is tracked for subsequent surgical steps thereon.

The interface 26 shows screen 1, in accordance with the progress that is made in the surgical procedure. Screen 1 may therefore display tracking information (e.g., axes, bone models, values) pertaining to the bone A from the tracking of the tibial reference 12. Moreover, according to the surgical procedure, screen 1 indicates that the next step is to connect the tibial digitizer 14A to the tibial reference 12, by way of the mating connection (e.g., Fig. 2) therebetween.

As mentioned previously, the positioning of the surgical device 14 may not require the physical interconnection with the surgical device 12. The data on the screen 1 may pertain to the installation of the surgical device 14 at a specific location on the bone A, per surgical technique, for instance at a given distance from the surgical device 12 as detectable by proximity switches and sensors.

Referring to Fig. 4B, the tibial digitizer 14A is matingly connected to the tibial reference 12, resulting in the trigger of the triggered unit 16. Accordingly, if the triggering conditions are met (e.g., time lapsed, proximity, etc.), the CAS processing unit 20 automatically changes the data on the interface 26, as indicated by screen 2 in Fig. 4B. The change of data may be the automatic change of mode in the procedure flow to start gathering data associated with the tibial digitizer 14A (e.g., the registration of a tibial axis), going from a commanding mode to a data-collecting mode. The change of data may also be an indication that the devices 12 and 14 are adequately connected to one another, thereby prompting the operator of the CAS system 10 to perform another step.

The aforementioned steps are part of a complete set of steps, some being mandatory or optional or prerequisites as defined in the surgical technique, operated by the CAS processing unit 20 in accordance with the CAS application 25. The aforementioned steps may be repeated during the surgical procedure. For instance, when installing the cutting block 14B (Fig. 3), the automatic trigger may also cause a change in the surgical procedure flow.

## Claims

1. A computer-assisted surgery system comprising:
a first surgical device (12) with a tracking unit mounted thereon and tracked during a surgical procedure and adapted to perform a first function associated to the surgical procedure;
a second surgical device (14A, 14B) adapted to perform a second function associated to the surgical procedure;
a triggered unit (16) triggered when the first surgical device and the second surgical device reach a predetermined proximity relation;
a surgical procedure processing unit (20) tracking at least the first surgical device, the surgical procedure processing unit (20) comprising:
a trigger detector (28) detecting a triggering of the triggered unit; a computer-assisted surgery application (25) operating steps of a surgical procedure;
a controller (24) for commanding the computer-assisted surgery application to activate a selected step associated with the second function when the trigger detector signals a detection; and
an interface (26) for displaying information about the selected step in the surgical procedure, **characterised in that** one of the surgical devices has a female connector (13), another of the surgical devices has a male connector (15A, 15B), whereby the surgical devices interconnect in a mating engagement of the male and female connectors.

2. The computer-assisted surgery system according to claim 1, wherein the triggered unit is a switch and the predetermined proximity relation is the mating engagement, whereby the switch is triggered by the mating engagement of the male and the female connector.

3. The computer-assisted surgery system according to any one of claims 1 or 2, wherein the first surgical device is a reference tool arranged to be mounted to a bone for tracking of the bone.

4. The computer-assisted surgery system according to claim 3, wherein the second surgical device is a digitizing tool releasably connected to the reference tool for determining an axis of the bone.

5. The computer-assisted surgery system according to any one of claims 1 to 4, further comprising a conditions database in one of the triggered unit and the trigger detector to confirm that the predetermined proximity relation is in accordance with conditions of the conditions database.

6. The computer-assisted surgery system according to any one of claims 1 to 5, wherein the next step is a change of mode, and the interface automatically changes mode when the trigger detector signals a detection.

7. The computer-assisted surgery system according to any one of claims 1 to 6, wherein the second surgical device has a tracking unit for being tracked during the surgical procedure.

8. The computer-assisted surgery system according to any one of claims 1 to 7, wherein the tracking unit comprises a micro-electromechanical system.

## Patentansprüche

1. Computerunterstütztes chirurgisches System, umfassend:
eine erste chirurgische Vorrichtung (12) mit einer Nachverfolgungseinheit, die daran montiert ist und während eines chirurgischen Vorgangs nachverfolgt wird, und die angepasst ist, um eine erste Funktion in Verbindung mit dem chirurgischen Vorgang durchzuführen;
eine zweite chirurgische Vorrichtung (14A, 14B), die angepasst ist, um eine zweite Funktion in Verbindung mit dem chirurgischen Vorgang durchzuführen;
eine ausgelöste Einheit (16), die ausgelöst wird, wenn die erste chirurgische Vorrichtung und die zweite chirurgische Vorrichtung eine vorgegebene Nähebeziehung erreichen;
eine chirurgische Vorgangs-Verarbeitungseinheit (20), die mindestens die erste chirurgische Vorrichtung nachverfolgt, wobei die chirurgische Vorgangs-Verarbeitungseinheit (20) Folgendes umfasst:
einen Auslösedetektor (28), der ein Auslösen der ausgelösten Einheit detektiert;
eine computerunterstützte chirurgische Anwendung (25), die Schritte eines chirurgischen Vorgangs bedient;
eine Steuerung (24) zum Befehlen der computerunterstützten chirurgischen Anwendung, einen ausgewählten Schritt in Verbindung mit der zweiten Funktion zu aktivieren, wenn der Auslösedetektor eine Detektion signalisiert; und
eine Schnittstelle (26) zum Anzeigen von Informationen über den ausgewählten Schritt im chirurgischen Vorgang,
**dadurch gekennzeichnet, dass** eine der chirurgischen Vorrichtungen einen Buchsenanschluss (13), eine andere der chirurgischen Vorrichtungen einen Steckeranschluss (15A, 15B) aufweist, wobei die chirurgischen Vorrichtungen in einem Passeingriff des Stecker- und Buchsenanschlusses untereinander verbunden werden.

2. Computerunterstütztes chirurgisches System nach Anspruch 1, wobei die ausgelöste Einheit ein Schalter ist und die vorgegebene Nähebeziehung der Passeingriff ist, wobei der Schalter durch den Passeingriff des Stecker- und Buchsenanschlusses ausgelöst wird.

3. Computerunterstütztes chirurgisches System nach einem der Ansprüche 1 oder 2, wobei die erste chirurgische Vorrichtung ein Referenzgerät ist, das angeordnet ist, um zum Nachverfolgen eines Knochens am Knochen montiert zu werden.

4. Computerunterstütztes chirurgisches System nach Anspruch 3, wobei die zweite chirurgische Vorrichtung ein Digitalisierungsgerät ist, das zum Bestimmen einer Achse des Knochens mit dem Referenzgerät lösbar verbunden ist.

5. Computerunterstütztes chirurgisches System nach einem der Ansprüche 1 bis 4, ferner umfassend eine Konditionendatenbank in einem der ausgelösten Einheit und des Auslösedetektors, um zu bestätigen, dass die vorgegebene Nähebeziehung im Einklang mit Konditionen der Konditionendatenbank steht.

6. Computerunterstütztes chirurgisches System nach einem der Ansprüche 1 bis 5, wobei der nächste Schritt eine Modusänderung ist und die Schnittstelle automatisch den Modus ändert, wenn der Auslösedetekor eine Detektion signalisiert.

7. Computerunterstütztes chirurgisches System nach einem der Ansprüche 1 bis 6, wobei die zweite chirurgische Vorrichtung eine Nachverfolgungseinheit aufweist, um während des chirurgischen Vorgangs nachverfolgt zu werden.

8. Computerunterstütztes chirurgisches System nach einem der Ansprüche 1 bis 7, wobei die Nachverfolgungseinheit ein mikro-elektromechanisches System umfasst.

## Revendications

1. Système de chirurgie assistée par ordinateur comprenant :
un premier dispositif chirurgical (12) sur lequel est montée une première unité de localisation qui est suivie pendant une intervention chirurgicale, et qui est adapté pour remplir une première fonction associée à l'intervention chirurgicale ;
un deuxième dispositif chirurgical (14A, 14B) adapté pour remplir une deuxième fonction associée à l'intervention chirurgicale ;
une unité déclenchée (16) qui est déclenchée lorsque le premier dispositif chirurgical et le deuxième dispositif chirurgical atteignent une relation de proximité prédéterminée ;
une unité de traitement d'intervention chirurgicale (20) qui suit au moins le premier dispositif chirurgical, l'unité de traitement d'intervention chirurgicale (20) comprenant :
un détecteur de déclenchement (28) qui détecte un déclenchement de l'unité déclenchée ;
une application de chirurgie assistée par ordinateur (25) qui exécute des étapes d'une intervention chirurgicale ;
un module de commande (24) pour commander l'application de chirurgie assistée par ordinateur pour activer une étape sélectionnée associée avec la deuxième fonction lorsque le détecteur de déclenchement signale une détection ; et
une interface (26) pour afficher des informations sur l'étape sélectionnée dans l'intervention chirurgicale,
**caractérisé en ce que** l'un des dispositifs chirurgicaux a un connecteur femelle (13), un autre des dispositifs chirurgicaux a un connecteur mâle (15A, 15B), grâce à quoi les dispositifs chirurgicaux s'interconnectent en un engagement par accouplement des connecteurs mâle et femelle.

2. Système de chirurgie assistée par ordinateur selon la revendication 1, dans lequel l'unité déclenchée est un contacteur et la relation de proximité prédéterminée est l'engagement par accouplement, dans lequel le contacteur est déclenché par l'engagement par accouplement des connecteurs mâle et femelle.

3. Système de chirurgie assistée par ordinateur selon l'une quelconque des revendications 1 ou 2, dans lequel le premier dispositif chirurgical est un outil de référence agencé pour être monté sur un os pour la localisation de l'os.

4. Système de chirurgie assistée par ordinateur selon la revendication 3, dans lequel le deuxième dispositif chirurgical est un outil de numérisation relié de manière libérable à l'outil de référence pour déterminer l'axe de l'os.

5. Système de chirurgie assisté par ordinateur selon l'une quelconque des revendications 1 à 4, comprenant en outre une base de données de conditions dans l'un de l'unité déclenchée et du détecteur de déclenchement pour confirmer que la relation de proximité prédéterminée est conforme aux conditions de la base de données de conditions.

6. Système de chirurgie assistée par ordinateur selon l'une quelconque des revendications 1 à 5, dans lequel l'étape suivante est un changement de mode, et l'interface change automatiquement de mode lorsque le détecteur de déclenchement signale une détection.

7. Système de chirurgie assistée par ordinateur selon l'une quelconque des revendications 1 à 6, dans lequel le deuxième dispositif chirurgical possède une unité de localisation pour être suivi pendant l'intervention chirurgicale.

8. Système de chirurgie assistée par ordinateur selon l'une quelconque des revendications 1 à 7, dans lequel l'unité de localisation comprend un système micro-électromécanique.
